# EUROPEAN PATENT SPECIFICATION

(11) **EP 2 841 685 B1**
(45) Date of publication and mention of the grant of the patent: **19.07.2017**
(21) Application number: 13781010.7
(22) Date of filing: 26.04.2013
(51) Int. Cl.: E21B 34/10, E21B 43/16, E21B 43/20

(54) **TUBING RETRIEVABLE INJECTION VALVE ASSEMBLY**
AUS EINEM ROHR ENTNEHMBARES EINSPRITZVENTIL
ENSEMBLE VANNE D'INJECTION RÉCUPÉRABLE DE PRODUCTION

(30) Priority: 27.04.2012 US 201261639569 P
(43) Date of publication of application: 04.03.2015
(73) Proprietor: Tejas Research and Engineering, L.P., The Woodlands, TX 77380 (US)
(72) Inventor: HILL, Thomas, G., Conroe, TX 77304 (US); HENSCHEL, Robert, C., The Woodlands, TX 77382 (US); HODGE, Rory, L., Spring, TX 77373 (US)
(74) Representative: Mansfield, Peter Turquand
(86) International application number: PCT/US2013/038438
(87) International publication number: WO 2013/163558

(56) References cited:
- US-A- 4 362 214
- US-A- 4 362 214
- US-A1- 2006 162 932
- US-A1- 2006 162 932

## Description

### BACKGROUND OF INVENTION

### 1. Field of the Invention

This invention is directed to an injection valve typically used in conjunction with an injection well. Injection wells are drilled for example in close proximity to producing oil or gas wells that have peaked in terms of their output. Fluid for example water is pumped under pressure into the injection well which in turn acts to force the oil or gas into the producing wells thus increasing the yield.

### 2. Description of Related Art

The provision of a valve assembly for use in an injection well to allow injection of fluids but to prevent reverse flow when injection is halted is described in US 2006/0162932 (McCalvin). During insertion of the valve assembly, a flapper valve is held open by a tubular lock; the valve assembly includes a flow restricting orifice, so that when fluid is injected into the well the pressure difference across the orifice slides a flow tube downward, pushing the lock downward, and compressing a spring; when flow ceases the spring pushes the flow tube back up, so the flapper valve is freed, and closes. US 4 362 214 (Pringle et al) describes a well safety valve for controlling outflow of fluid from a well; this includes a flow tube pushed down by a spring, the flow tube including a choke to restrict flow; the flow tube prevents a flap valve from closing, but if the flow rate becomes too great the flow tube is pushed up, compressing the spring, and freeing the flap valve, which closes. In this case the choke bean is replaceable without removing the valve assembly.

### BRIEF SUMMARY OF THE INVENTION

The invention includes providing a tubing retrievable injection valve having a full bore internal diameter when running and retrieving the valve. A slick line retrievable choke is carried by a retrievable orifice selective lock assembly. The choke is retrievable without removing the injection valve. Consequently the diameter of the choke orifice may be changed on the surface. The injection valve also has a temporary lock out feature so that the valve may be placed in the well in a lock out mode. The invention is defined in the claims.

### BRIEF DESCRIPTION OF THE SEVERAL VIEWS OF THE DRAWING(S)

FIG. 1 is a cross sectional view of the valve in a lock out, running position.
FIG. 2 is a cross sectional view of the valve in a pre-injection position with the valve member closed.
FIG. 3 is a cross-sectional view of the retrievable orifice selective lock assembly.
FIG. 4 is a perspective view of the retrievable orifice selective lock assembly.
FIG. 5 is a cross sectional view of the valve showing the retrievable orifice selective lock assembly located within the valve body.
FIG. 6 is a cross sectional view of the valve in an open injection position.

### DETAILED DESCRIPTION OF THE INVENTION

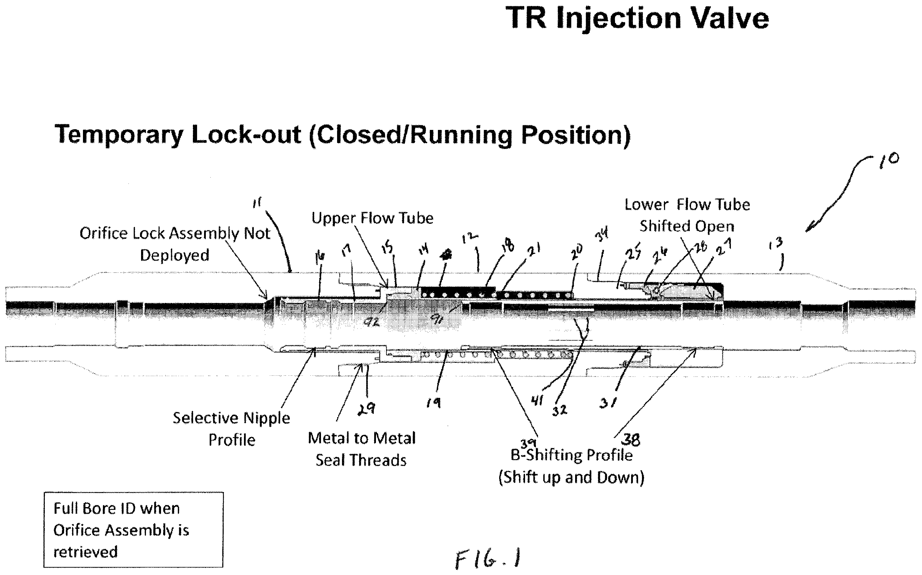

Referring to FIG. 1, an embodiment of the invention includes a pressure containing body comprising an upper valve body member 11 to a tubular, a middle valve body member 12 suitably attached to the upper valve body member 11 by threads at 29, for example, and a lower valve body member 13 which is connectable to a tubular at its downhole end. Valve body members 12 and 13 are secured to each other by threads for example at 34.

The injection valve 10 further includes an upper flow tube 15 having a first section 17 and a second section 14 which are secured together. Section 17 has an interior nipple profile at 16 for receiving a tool. Section 14 has an elongated sleeve portion 19 that extends to valve seat 26 when the valve is in the position shown in FIG. 1. A lower flow tube 31 is positioned within the elongated sleeve portion 19 of the upper flow tube. Lower flow tube 31 has a plurality of slots 32 and a plurality of ridges formed on its outer periphery where the slots 32 are located so as to form a flexible profile for engagement with recesses 21 and 20 on the inner surface of elongated sleeve portion 19. Lower flow tube 31 also has shifting profiles 39 and 38 at each end thereof.

Middle body member 12 has a reduced diameter portion 25 that carries an annular valve seat 26. A flapper valve 27 is pivotably connected at 28 to portion 25 and is resiliently biased to a closed position on valve seat 26 as is known in the art.

A coil spring 18 is positioned about elongated sleeve portion 19 and is captured between shoulder 14 of the upper flow tube and an internal shoulder 41 provided within middle valve body member 12.

In the temporary lock out running position shown in FIG. 1, lower flow tube 31 is positioned within the valve body so as to extend beyond valve seat 26 thereby maintaining flapper valve 27 in an open position.

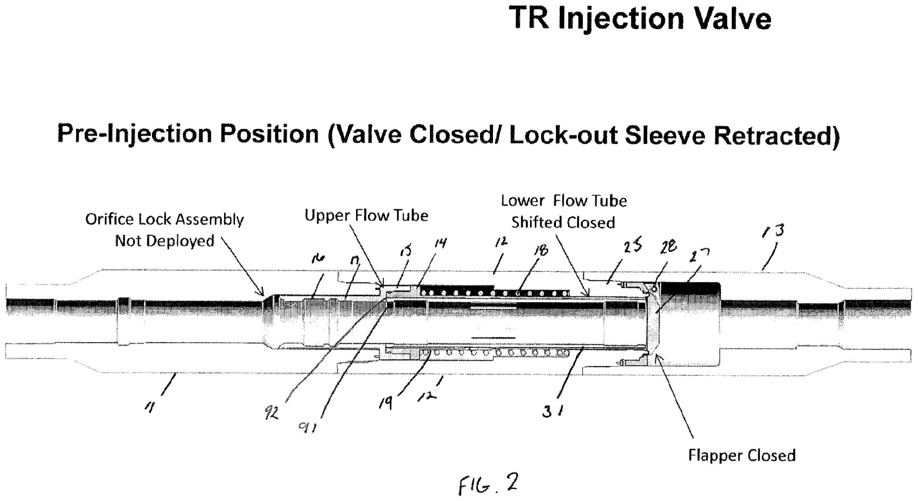

When the valve is positioned within the well at the desired location, a suitable running tool is lowered into the well and engages the upper shifting profile 39 of lower flow tube 31 and the flow tube is moved upwardly or to the left as shown in FIG. 1, to the position shown in FIG. 2. The uphole end portion 91 of the lower flow tube 31 will abut a shoulder portion 92 of the upper flow tube 15 as shown in FIG. 2. In this position, the resiliently biased flapper valve 27 will be in the closed position.

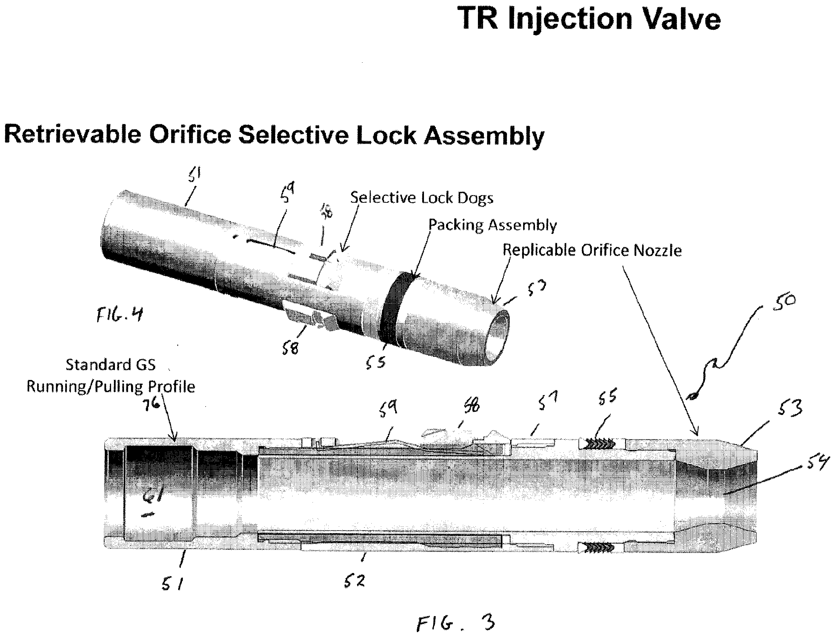

The retrievable orifice selective lock assembly (ROSLA) will now be discussed with reference to FIGs. 3 and 4. The ROSLA 50 includes a shiftable sleeve formed by generally cylindrical member 51, 52, and 53 having an interior flow passage 61. A plurality of selective lock dogs 58 are located around a portion of its periphery as shown in FIG. 4. Leaf springs 59 are positioned under lock dogs 58. ROSLA 50 includes an annular packing assembly 55. A replaceable and retrievable orifice nozzle 53 is releasably attached to the body portion of the ROSLA and includes an orifice 54. Orifice nozzle 53 may be replaced on the surface with another nozzle having a different size orifice 54.

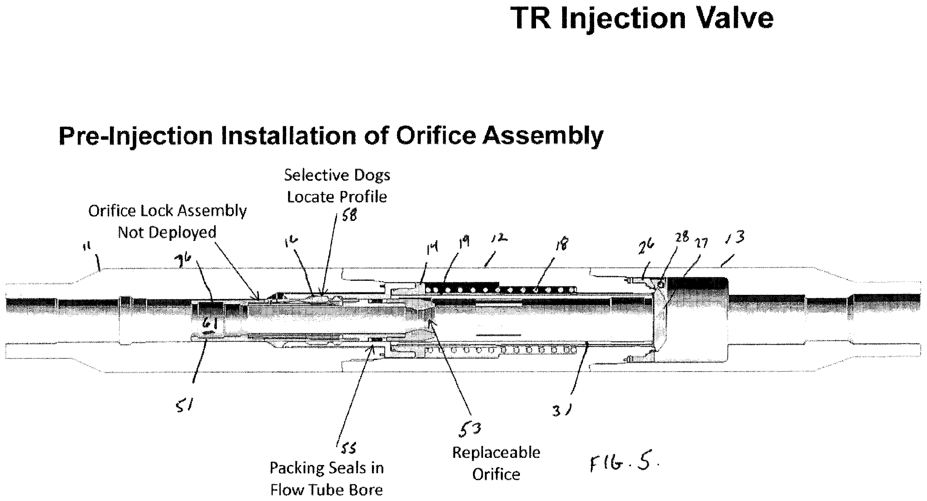

FIG. 5 illustrates the position of the ROSLA within the injection valve prior to the injection stage. ROSLA may be lowered into the valve body by a suitable tool to a position where the selective lock dogs 58 engage the selective nipple profile 16 in the first section 17 of the upper flow tube 15. At this point the ROSLA will be physically connected to the upper flow tube 15; however flapper valve 27 is still in the closed position.

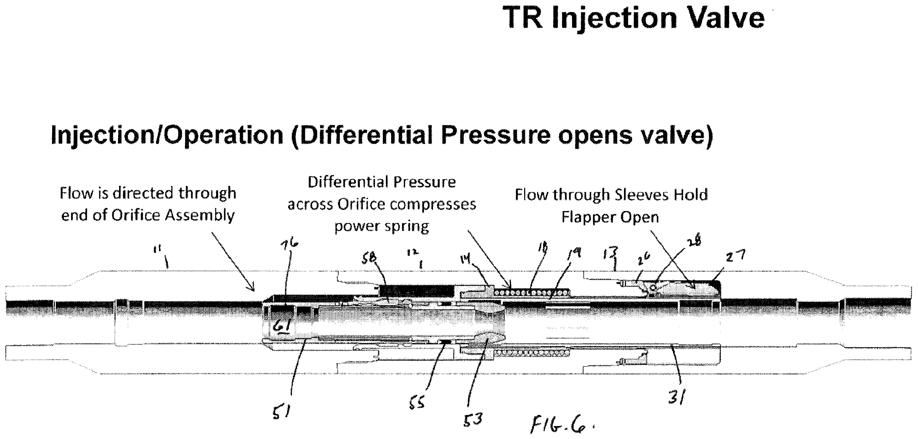

The next step in the process is to pump a fluid such as water under pressure into the valve body. As the fluid flows through the ROSLA, a pressure drop will occur across orifice 54 which will cause the ROSLA and upper flow tube assembly 15, 14 to move downhole or to the right as shown in FIG. 6.

This movement will compress spring 18. The downhole portions of both the upper flow tube 15 and lower flow tube 31 will be forced into contact with flapper valve 27 and as they are moved further by the pressure differential, they will open the flapper valve to the position as shown in FIG. 6.

As long as the fluid is being pumped the injection valve will remain open. However when the pumping stops, compressed spring 18 will move the ROSLA and the upper and lower flow tubes 15 and 31 back to the position shown in FIG. 5 in which the flapper valve 27 is in the closed position.

Although the present invention has been described with respect to specific details, it is not intended that such details should be regarded as limitations on the scope of the invention, except to the extent that they are included in the accompanying claims.

## Claims

1. A method of injecting fluid into a well comprising:
providing a valve assembly having a temporary lock out mode, an upper flow tube (15) and lower flow tube (31), a flapper valve (27) and a valve seat (26),
placing the valve assembly in a temporary lock out mode, by positioning the lower flow tube (31) to extend beyond the valve seat (26) thereby maintaining the flapper valve (27) in an open position;
positioning the valve assembly on a tubing and running the tubing into the well, injecting fluid into the valve assembly thereby opening the flapper valve (27), and
closing the flapper valve (27) by termination the flow of fluid into the valve assembly
***characterized in that*** the valve assembly also comprises a retrievable orifice selective lock assembly (50), and **in that** the lower flow tube (31) defines a shifting profile (38, 39) and is positioned within an elongated sleeve portion (19) of the upper flow tube (15),
and **in that**, prior to injecting the fluid into the valve assembly, the method comprises:
shifting the lower flow tube (31) to a retracted position within the sleeve portion (19) by means of the shifting profile (38, 39), and then
running the retrievable orifice selective lock assembly (50) into the valve assembly.

2. An injection valve (10) for use in a subterranean well comprising: a pressure containing body (11, 12, 13) attachable to a production tubing, a shiftable sleeve comprising an upper flow tube (15) and a lower flow tube (31), the sleeve being shiftable by means of an orifice (53), where said shiftable sleeve operates in response to fluid being injected into the well from the surface, and the injection valve (10) comprises a closure means (27, 26),
***characterised in that*** the lower flow tube (31) is positioned within an elongated sleeve portion (19) of the upper flow tube (15), and defines a shifting profile (38, 39) so it can be moved axially and can be retracted into the sleeve portion (19) ;
and **in that** the orifice (53) is provided by a retrievable orifice selective lock assembly (50) which is positionable within the valve body.

3. The injection valve of claim 2 wherein said retrievable orifice assembly (50)
may be retrieved from the wellbore while leaving said closure means (27, 26) in the well in a closed position.

4. The injection valve of claim 2 wherein said shiftable sleeve (31) is adapted to
be alternately shifted to a first position to lock the injection valve in the open position.

## Patentansprüche

1. Verfahren zur Einspritzung eines Fluids in ein Bohrloch umfassend:
Bereitstellen einer Ventilanordnung, die einen vorübergehenden Festlegemodus, eine oberes Durchflussrohr (15) und ein unteres Durchflussrohr (31), eine Luftklappe (27) und einen Ventilsitz (26) hat,
Einstellen der Ventilanordnung in einen vorübergehenden Festlegemodus mittels Positionierung des unteren Durchflussrohres (31) so, dass es sich jenseits des Ventilsitzes (26) erstreckt, wodurch die Luftklappe (27) in einer offenen Position verbleibt;
Positionieren der Ventilanordnung auf einer Rohrleitung und Einführen der Rohrleitung in das Bohrloch, Einspritzen von Fluid in die Ventilanordnung, wodurch die Luftklappe (27) geöffnet wird, und
Schließen der Luftklappe (27) durch Beenden des Flusses von Fluid in die Ventilanordnung
**dadurch gekennzeichnet, dass** die Ventilanordnung auch eine abrufbare Öffnungselektivfestlegeanordnung (50) umfasst,
und dadurch, dass das untere Durchflussrohr (31) ein Verlagerungsprofil (38, 39) festlegt und in einem gestreckten Hülsenabschnitt (19) des oberen Durchflussrohres (15) positioniert ist,
und dadurch, dass vor Einspritzung des Fluids in die Ventilanordnung, das Verfahren umfasst:
Verlagerung des unteren Durchflussrohres (31) in eine zurückgezogene Position innerhalb des Hülsenabschnitts (19) mittels des Verlagerungsprofils (38, 39), und dann
Einführen der abrufbaren Öffnungselektivfestlegeanordnung (50) in die Ventilanordnung.

2. Einspritzventil (10) zur Verwendung in einem unterirdischen Bohrloch umfassend:
einen Druck enthaltenden Körper (11, 12, 13), der an einer Produktionsrohrleitung anbringbar ist, eine verlagerbare Hülse umfassend ein oberes Durchflussrohr (15) und ein unteres Durchflussrohr (31), wobei die Hülse mittels einer Öffnung (53) verlagerbar ist, wobei die verlagerbare Hülse in Antwort auf in das Bohrloch von der Oberfläche eingespritztes Fluid operiert, und wobei das Einspritzventil (10) Verschlussmittel (27, 26) umfasst,
**dadurch gekennzeichnet, dass** das untere Durchflussrohr (31) innerhalb eines gestreckten Hülsenabschnitts (19) des oberen Durchflussrohres (15) positioniert ist, und ein Verlagerungsprofil (38, 39) festlegt, so dass es axial bewegt werden kann, und in den Hülsenabschnitt (19) zurückgezogen werden kann;
und dadurch, dass die Öffnung (53) mittels einer abrufbaren Öffnungselektivfestlegeanordnung (50) bereitgestellt ist, die innerhalb des Ventilkörpers positionierbar ist.

3. Einspritzventil nach Anspruch 2, wobei die abrufbare Öffnungsanordnung (50) aus der Bohrlochbohrung zurückgeholt werden kann, während die Verschlussmittel (27, 26) in dem Bohrloch in einer verschlossenen Position verbleiben.

4. Einspritzventil nach Anspruch 2, wobei die verlagerbare Hülse (31) geeignet ist, abwechselnd in eine erste Position zum Festlegen des Einspritzventils in der offenen Position verlagert zu werden.

## Revendications

1. Procédé d'injection de fluide dans un puits comprenant :
la fourniture d'un ensemble soupape possédant un mode de verrouillage temporaire, un tube d'écoulement supérieur (15) et un tube d'écoulement inférieur (31), une soupape à languette (27) et un siège de soupape (26),
la mise en place de l'ensemble soupape dans un mode de verrouillage temporaire, en positionnant le tube d'écoulement inférieur (31) pour s'étendre au-delà du siège de soupape (26) ce qui permet de maintenir la soupape à languette (27) dans une position ouverte ;
le positionnement de l'ensemble soupape sur un tubage et le passage du tubage dans le puits, l'injection de fluide dans l'ensemble soupape ouvrant de ce fait la soupape à languette (27), et
la fermeture de la soupape à languette (27) par achèvement de l'écoulement de fluide dans l'ensemble soupape
**caractérisé en ce que** l'ensemble soupape comprend également un ensemble orifice récupérable à verrouillage sélectif (50),
et **en ce que** le tube d'écoulement inférieur (31) définit un profil de déplacement (38, 39) et est positionné au sein d'une partie de manchon allongée (19) du tube d'écoulement supérieur (15),
et **en ce que**, avant injection du fluide dans l'ensemble soupape, le procédé comprend :
le déplacement du tube d'écoulement inférieur (31) à une position rétractée au sein de la partie de manchon (19) au moyen du profil de déplacement (38, 39), puis
le passage de l'ensemble orifice récupérable à verrouillage sélectif (50) dans l'ensemble soupape.

2. Soupape d'injection (10) destinée à être utilisée dans un puits souterrain, comprenant :
un corps renfermant une pression (11, 12, 13) pouvant être fixé à un tubage de production, un manchon déplaçable comprenant un tube d'écoulement supérieur (15) et un tube d'écoulement inférieur (31), le manchon étant déplaçable au moyen d'un orifice (53), où ledit manchon déplaçable fonctionne en réponse à un fluide étant injecté dans le puits depuis la surface, et la soupape d'injection (10) comprend un moyen de fermeture (27, 26),
**caractérisée en ce que** le tube d'écoulement inférieur (31) est positionné au sein d'une partie de manchon allongée (19) du tube d'écoulement supérieur (15), et définit un profil de déplacement (38, 39) de sorte qu'il peut être déplacé axialement et peut être rétracté dans la partie de manchon (19) ;
et **en ce que** l'orifice (53) est fourni par un ensemble orifice récupérable à verrouillage sélectif (50) qui est apte à être positionné au sein du corps de soupape.

3. Soupape d'injection selon la revendication 2, dans laquelle ledit ensemble orifice récupérable (50) peut être récupéré du puits de forage tout en laissant ledit moyen de fermeture (27, 26) dans le puits dans une position fermée.

4. Soupape d'injection selon la revendication 2, dans laquelle ledit manchon déplaçable (31) est conçu pour être déplacé en alternance à une première position pour verrouiller la soupape d'injection dans la position ouverte.
